# EUROPEAN PATENT APPLICATION

(11) **EP 4 763 067 A1**
(43) Date of publication of application: **24.06.2026**
(21) Application number: 25218634.1
(22) Date of filing: 26.11.2025
(51) Int. Cl.: A61B 5/024, A61B 5/344, A61B 5/391, A61B 5/00, A61B 8/08

(54) **MATERNAL AND FETAL MONITORING SYSTEMS**

(30) Priority: 13.12.2024 US 202418980419
(71) Applicant: GE Precision Healthcare LLC, Waukesha, WI 53188 (US)
(72) Inventor: MANICKAM, Kalaivani, Waukesha, 53188 (US); NAIK, Rajendra, Waukesha, 53188 (US); KAVOORI SETHUMADHAVAN, Nagapriya, Waukesha, 53188 (US)
(74) Representative: Kilburn & Strode LLP

(57) **Abstract**

A patient monitoring system includes a set of electrodes configured to obtain a uterine activity (UA) signal and a total abdominal electrical signal from an abdomen of a maternal patient, wherein the total abdominal electrical signal includes at least the UA signal, a maternal heart signal, and a fetal heart signal. At least a subset of the set of electrodes is configured as a low pass filter to obtain the UA signal. A patient monitor is configured to subtract the UA signal from the total abdominal signal to generate a total heart signal containing at least the maternal heart signal and the fetal heart signal, determine a maternal heart rate based on the total heart signal, and output the UA signal and the maternal heart rate.

## Description

### BACKGROUND

The present disclosure generally relates maternal and fetal monitoring, and specifically to a device and method for monitoring maternal and fetal heart rates and maternal uterine activity.

Prior to the onset of labor, a pregnant patient prefers to be ambulatory. In other words, the pregnant patient prefers to be able to move about freely, whether in the patient's own home or within the hospital. However, a pregnant patient who is likely to begin labor soon has reduced ambulatory ability due to the number of sensors that are normally attached to their abdomen to monitor both the onset of labor and the health of the unborn baby.

Sensors are often attached to a pregnant patient during pre-labor and intra labor for monitoring the fetal heart rate (fHR) and uterine activity (i.e., maternal contractions). Additionally, maternal heart rate is another important parameter to monitor maternal physiological parameters apart from fetal health. Various sensor arrangements and monitoring systems are available for tracking fetal heart rate (fHR), uterine activity (UA), and maternal heart rate (mHR). For example, systems are known that are configured to detect a fetal electrocardiogram (FECG) and/or fHR without making physical contact with the fetus. For example, some monitoring systems use electrodes configured to be placed on the mother's skin about the abdomen to detect electro physiological signals. Uterine activity can be determined from the electrophysiological signals, such as electromyography (EMG) signals. One example of such a system is the Novii wireless patch system available from GE Healthcare.

### SUMMARY

This Summary is provided to introduce a selection of concepts that are further described below in the Detailed Description. This Summary is not intended to identify key or essential features of the claimed subject matter, nor is it intended to be used as an aid in limiting the scope of the claimed subject matter.

In one aspect of the disclosure, a patient monitoring system includes a set of electrodes configured to obtain a uterine activity (UA) signal and a total abdominal electrical signal from an abdomen of a maternal patient, wherein the total abdominal electrical signal includes at least the UA signal, a maternal heart signal, and a fetal heart signal. At least a subset of the set of electrodes is configured as a low pass filter to obtain the UA signal. A patient monitor is configured to subtract the UA signal from the total abdominal signal to generate a total heart signal containing at least the maternal heart signal and the fetal heart signal, determine a maternal heart rate based on the total heart signal, and output the UA signal and the maternal heart rate.

In certain embodiments, at least one of the set of electrodes configured to act as the low pass filter may be configured to only pass frequencies below a threshold frequency. In certain embodiments, the threshold frequency is between 0.1 Hz and 2 Hz.

In certain embodiments, at least one of the electrodes in the set of electrodes may have a width in the range of 4 cm to 20 cm such that it is configured to act as the low pass filter.

In certain embodiments, at least one of the electrodes in the set of electrodes may have a width of at least 10 cm such that it is configured to act as the low pass filter.

In certain embodiments, at least one of the electrodes in the set of electrodes may have a width of at least 15 cm such that it is configured to act as the low pass filter.

In certain embodiments, at least one of the electrodes in the set of electrodes may have a width of at least 20 cm such that it is configured to act as the low pass filter.

In certain embodiments, at least one of the electrodes in the set of electrodes may be a printed electrode and may include at least one printed circuit element configured to act as the low pass filter.

In certain embodiments, the set of electrodes may include at least three electrodes comprising a one electrode configured in a monopolar arrangement to obtain the UA signal, that may be configured to only pass frequencies below a threshold frequency. The second and third electrodes may be configured in a bipolar arrangement to obtain the total abdominal electrical signal from the maternal patient.

In certain embodiments, the first electrode has a width of at least 15 cm.

In certain embodiments, the first electrode has a width of at least 20 cm.

In certain embodiments, the first electrode may have a width of at least 15 cm and at least one of the second electrode and the third electrode may have a width less than 2 cm.

In certain embodiments, the first electrode, the second electrode, and/or the third electrode may be mounted on a substrate comprising a patch configured to adhere to the abdomen of the maternal patient. In certain embodiments, the patch may be configured such that the second electrode and the third electrode are movable with respect to the first electrode.

In certain embodiments, the set of electrodes may be an electrode array mounted on a substrate comprising a patch configured to adhere to the abdomen of the maternal patient. The electrode array may be configured to output multiple signals that are utilized to generate the low frequency UA signal.

In certain embodiments, the electrode array may comprise at least one linear array of at least 4 electrodes arranged in a line.

In certain embodiments, the electrode array may comprise at least one linear array of at least 6 electrodes arranged in a line.

In certain embodiments, the electrode array may comprise at least one linear array of at least 8 electrodes arranged in a line.

In certain embodiments, the electrode array may comprise at least one linear array of at least 10 electrodes arranged in a line.

In certain embodiments, the electrode array may comprise a grid, wherein the grid is at least 2 electrodes wide and at least 2 electrodes high

In certain embodiments, the electrode array may comprise a grid, wherein the grid is at least 6 electrodes wide and at least 3 electrodes high.

In certain embodiments, each electrode in the electrode array may be separated by at least 4 mm.

In certain embodiments, the system may comprise an ultrasound transducer configured to be adhered to the maternal abdomen and to obtain the fetal heart signal. The patient monitor may be configured to determine the maternal heart rate by subtracting the fetal heart signal from the total heart signal.

In one aspect of the disclosure, a set of electrodes is configured for abdominal detection of maternal electrophysiological signals, such as electromyographical signals. The set of electrodes comprises at least a first electrode configured to record a uterine activity (UA) signal from an abdomen of a maternal patient, wherein the at least the first electrode is configured as a low pass filter to obtain the UA signal. At least a pair of electrodes is configured in a bipolar arrangement to record a total abdominal electrical signal from the maternal patient, wherein the total abdominal electrical signal includes at least the UA signal, a maternal heart signal, and a fetal heart signal.

In certain embodiments, the first electrode has a width of at least 15 cm such that it is configured to act as the low pass filter to remove frequencies below a threshold, such as below 2 Hz.

In certain embodiments, the first electrode has a width of at least 15 cm, and at least one of the second electrode and the third electrode may have a width less than 2 cm.

In certain embodiments, a plurality of electrodes is configured to act as a low pass filter to record the UA signal, wherein the plurality of electrodes may comprise an electrode array or an electrode grid comprising at least 4 electrodes mounted on a substrate comprising a patch configured to adhere to the abdomen of the maternal patient. The electrode array or grid is configured to output multiple signals that are utilized together to generate the low frequency UA signal. For example, the multiple signals may be added together or averaged, or otherwise combined using linear operations, to generate the low frequency UA signal.

In one embodiment, the electrode array or grid is further configured to obtain and output a total abdominal electrical (TAE) signal from the maternal patient.

Optionally, the one or many of the multiple signals may be adaptively selected and used in monopolar or bipolar mode and combined using adding, averaging or other linear operations, to generate the TAE.

Various other features, objects, and advantages of the invention will be made apparent from the following description taken together with the drawings.

### BRIEF DESCRIPTION OF THE DRAWINGS

The present disclosure is described with reference to the following Figures.
FIG. 1 is a schematic diagram of an exemplary embodiment of a maternal and fetal monitoring system.
FIG. 2 is a schematic diagram of another exemplary embodiment of a maternal and fetal monitoring system.
FIG. 3 is an environmental view including an exemplary embodiment of a maternal and fetal monitoring system according to the present disclosure.
FIG. 4 is a graph showing an exemplary total abdominal electrical signal obtained with an exemplary electrode arrangement according to one embodiment of the present disclosure.
FIG. 5 is a graph showing an exemplary uterine activity signal obtained with the exemplary electrode arrangement according to one embodiment of the present disclosure.
FIG. 6 is a graph showing an exemplary total heart signal obtained by removing the uterine activity signal of FIG. 4 from the total abdominal electrical signal of FIG. 5.
FIG. 7 shows one embodiment of a set of electrodes according to the present disclosure.
FIG. 8 shows another embodiment of a set of electrodes according to the present disclosure.
FIG. 9 shows another embodiment of a set of electrodes according to the present disclosure.
FIG. 10 shows another embodiment of a set of electrodes according to the present disclosure.
FIG. 11 shows another embodiment of a set of electrodes according to the present disclosure.
FIG. 12 shows another embodiment of a set of electrodes according to the present disclosure.
FIG. 13 shows another embodiment of a set of electrodes according to the present disclosure.
FIG. 14 shows another embodiment of a set of electrodes according to the present disclosure.
FIGS. 15A-17B show embodiments of electric circuits for an electrode configured as a low pass filter according to embodiments of the present disclosure.

### DETAILED DESCRIPTION

In the present description, certain terms have been used for brevity, clarity and understanding. No unnecessary limitations are to be inferred therefrom beyond the requirement of the prior art because such terms are used for descriptive purposes only and are intended to be broadly construed.

As used herein, unless otherwise limited or defined, discussion of particular directions is provided by example only, with regard to particular embodiments or relevant illustrations. For example, discussion of "top," "bottom," "front," "rear," "left," "right," "horizontal," "vertical," and "longitudinal" features and/or relative motion, e.g., movement "up" and "down," is generally intended as a description only of the orientation of such features relative to a reference frame of a particular example or illustration. Correspondingly, for example, a "top" feature may sometimes be disposed below a "bottom" feature (and so on), in some arrangements or embodiments. Additionally or alternatively, embodiments may be arranged in a different orientation such that "top" and "bottom" features are arranged horizontally relative to each other, for example in a "left-to-right" orientation.

The use herein of the terms "including," "comprising," or "having," and variations thereof, is meant to encompass the elements listed thereafter and equivalents thereof, as well as additional elements. Embodiments recited as "including," "comprising," or "having" certain elements are also contemplated as "consisting essentially of" and "consisting of" those certain elements.

As used herein, the terms controller or module may refer to, be part of, or include an application-specific integrated circuit (ASIC), an electronic circuit, a combinational logic circuit, a field programmable gate array (FPGA), a processor (shared, dedicated, or group) that executes code, or other suitable components that provide the described functionality, or a combination of some or all of the above, such as in a system-on-chip. The terms controller or module may include memory (shared, dedicated, or group) that stores code executed by the processor. The term code, as used herein, may include software, firmware, and/or microcode, and may refer to programs, routines, functions, classes, and/or objects. The term shared, as used above, means that some or all code from multiple modules may be executed using a single (shared) processor. In addition, some or all code to be executed by multiple different processors may be stored by a single (shared) memory. The term group, as used above, means that some or all code comprising part of a single controller or module may be executed using a group of processors. Likewise, some or all code comprising a single controller or module may be stored using a group of memories.

Aspects of the disclosure are described herein in terms of functional and/or logical block components and various processing steps. It should be appreciated that such block components may be realized by any number of hardware, software, and/or firmware components configured to perform the specified functions. For example, an embodiment may employ various integrated circuit components, e.g., memory elements, digital signal processing elements, logic elements, look-up tables, or the like, which may carry out a variety of functions under the control of one or more processors or other control devices. In addition, those skilled in the art will appreciate that the present invention may be practiced in conjunction with any number of medical devices, including any number of different physiological data acquisition devices, and that the system described herein is merely one example application. The connecting lines shown in the various figures contained herein are intended to represent example functional relationships and/or physical couplings between the various elements. It should be noted that many alternative or additional functional relationships or physical connections may be present in a practical embodiment.

The inventors have recognized that current monitoring systems with integrated monitoring electrodes for obtaining both fetal cardiac activity and maternal cardiac activity along with uterine activity (UA) are not ideal, particularly where two or more fetuses are present (i.e., twins, triplets, etc.). Thus, clinicians often prefer to utilize ultrasound for non-invasively obtaining fetal heart rate (fHr). However, existing electrode systems for detecting UA and maternal heart rate (mHr) are cumbersome and not configured to be easily used in conjunction with ultrasound monitor(s). Existing multi-electrode systems for UA and mHr cover much of the abdomen and are not configured to accommodate also attaching an ultrasound transducer to the maternal abdomen. An ultrasound transducer, when used to obtain the fHr, typically needs to be moved around to locate a strong fetal heart rate signal and may need to be moved several times throughout a measurement period. So a large patch that covers substantial sections of the lower abdomen interferes with the use of the ultrasound transducer to measure fHr. Sometimes tocodynamometers are utilized for obtaining UA; however, tocodynamometers are large. Two bulky transducers attached to the maternal abdomen using belts is uncomfortable for the mother. Moreover, measuring UA by tocodynamometry suffers from challenges because it relies on external pressure changes which are influenced by many external and internal factors. Toco transducers are susceptible to interference from maternal movement or shifting of the abdominal transducer. Tocodynamometry can be especially unreliable on obese patients, where the abdominal fat separates the tocodynamometer from the uterus, and in the second stage of labor when there is often considerable maternal movement.

Accordingly, the inventors have developed the disclosed patient monitoring system and electrode set configured to measure UA and mHr and configured to be worn on the maternal abdomen with an ultrasound transducer configured to measure fHr. The electrode set is configured such that it does not obscure the lower portion of the maternal abdomen, where the ultrasound transducer may be placed, and thus is configured to be adhered at locations such as within the top half of the maternal abdomen. At least a subset of the set of electrodes is configured as a low pass filter to obtain the UA signal, such as to only pass frequencies below a threshold frequency associated with UA. In one embodiment, at least one of the set of electrodes is larger than the other electrode(s) such that it acts as a low pass filter for obtaining the UA signal. For example, the at least one larger electrode configured to act as a low pass filter has a width of at least 10 cm or at least 15cm and is configured to be placed lengthwise across the width of the maternal abdomen, whereas the at least one electrode configured to obtain the total abdominal electrical (TAE) signal may be less than 2 cm wide. In some embodiments, the large electrode(s) configured for low pass filtering may be at least 18 cm wide, or at least 20 cm wide. In other embodiments, one or more of the electrodes configured to obtain UA may include printed circuit elements configured to filter the signal, such as capacitive and resistive elements.

In still other embodiments, the set of electrodes may include an electrode grid configured as an electrode array mounted on a substrate forming a patch configured to adhere to the abdomen of the maternal patient, wherein the electrode array is configured to output multiple signals that are combined together, such as added or averaged, to generate the low frequency UA signal. The electrode array may include several electrodes, such as more than 10 electrodes or more than 20 electrodes arranged in one or more linear arrays and configured to provide outputs from multiple sets of two electrodes (which may be two electrodes in the grid or single electrodes in the grid all referenced to a reference electrode that is outside of the grid). The outputs are then combined together, such as by hardware amplifiers and/or logic gates or using software methods, such that the low frequency signal common to all the sets of electrodes results. For example, the outputs may be added together or averaged, or otherwise combined using linear operations.

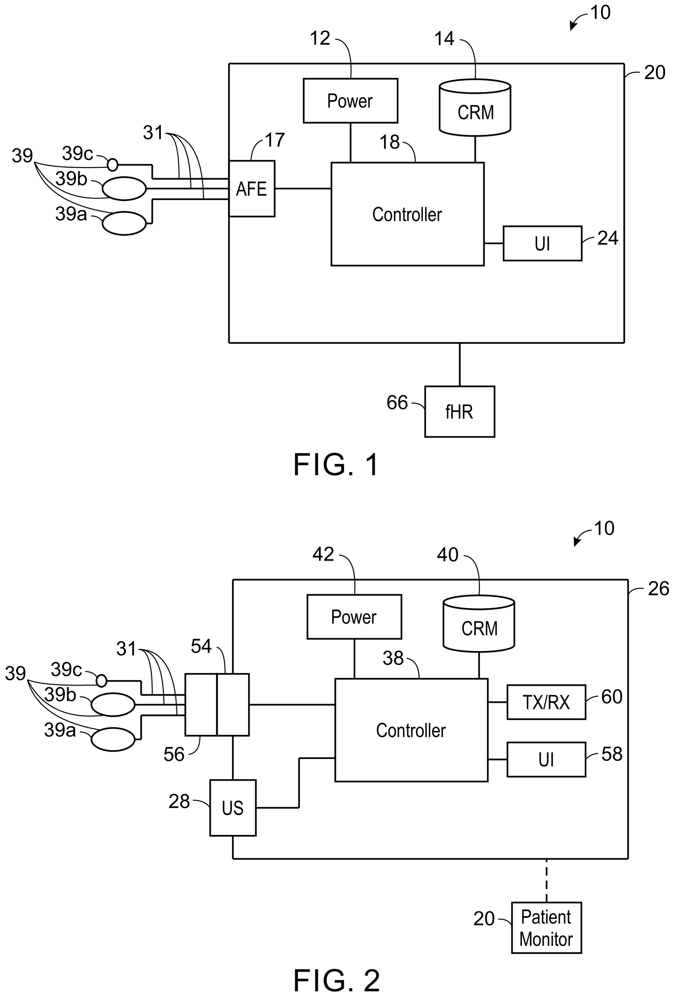

FIG. 1 is a schematic diagram of an exemplary embodiment of a maternal and fetal monitoring system 10 comprising a set of electrodes 39 configured to record a UA signal from an abdomen of a maternal patient, wherein at least one of the plurality of electrodes 39a-39c is configured as a low pass filter to obtain the UA signal. The electrode set 39 includes at least three electrodes, and in this embodiment includes an electrode 39a configured to only pass signals below a threshold frequency so as to obtain the UA signal. A second electrode 39b and a third electrode 39c, at least one of which may be smaller than the first electrode, are configured to obtain the total abdominal electrical (TAE) signal. Numerous other embodiments for the set of electrodes 39 are described herein. In the illustrated embodiment, the first electrode 39a and the second electrode 39b are depicted as being generally the same size as one another. As such, the first electrode 39a and the second electrode 39b of FIG. 1 are both configured such that they may be used by the system 10 as a low-pass electrode. Some embodiments, however, may be differently configured. For example, some embodiments of an electrode set 39 may include one large electrode *(e.g.,* the first electrode 39a of FIG. 1) for obtaining the UA signal and a pair of smaller electrodes (e.g., the third electrode 39c of FIG. 1) for obtaining the TAE signal.

A patient monitor 20 is configured to receive the UA signal and TAE signal from the set of electrodes 39 and comprises a power source 12. For example, the power source 12 may exemplarily be configured as a battery, such as a rechargeable battery. Some embodiments, however, may be differently configured. An analog front end (AFE) 17 is configured to digitize the both the UA and TAE signals. In one embodiment, the patient monitor 20 includes a controller 18 configured to execute software to determine a total cardiac signal (which comprises both the maternal heart signal and the fetal heart signal) by removing the UA signal from the TAE signal. The UA signal may be removed from the TAE signal by subtracting the UA signal from the TAE signal, and/or by using other linear and/or non-linear processing methods. Additionally or alternatively, the patient monitor may include analog circuitry configured to subtract (or otherwise remove) the UA signal from the TAE signal, and thus to generate the total cardiac signal by analog means.

The patient monitor 20 is configured to determine the maternal heart rate (mHR) based on the total cardiac signal. In the depicted embodiment, the monitoring system 10 includes a fetal heart rate monitor 66 configured to measure physiological signals. In one embodiment, the fetal heart rate monitor 66 is an ultrasound transducer configured to acquire doppler ultrasound signals from the exterior surface of the maternal abdomen and to determine the fetal heart rate (fHR) based thereon. In other embodiments, the fetal heart monitor may include other non-invasive means for determining fHR, or may include more invasive means for acquiring fetal heart signals, such as fetal ECG electrodes configured to be placed on the scalp of the fetus while it is in the maternal abdomen. In other embodiments, the controller 18 may be configured to execute signal processing software to separate each of the maternal heart signal and the fetal heart signal from the total heart (TH) signal.

Communication between the fHR monitor 66 and/or the electrodes 39a, 39b, 39c and the patient monitor 20 may be conducted via any analog or digital communication means. In some embodiments in which the fHR monitor 66 and/or the electrodes 39a, 39b, 39c communicate wirelessly with the patient monitor 20, the maternal and fetal monitoring system 10 may include a communication module (not shown) with an analog front end and a transceiver configured to communicate the fHR (and/or the entire maternal heart signal) or the UA and TAE signals to the patient monitor 20. The communication module may be connected to the electrode set 39 and may be mounted on the maternal abdomen and configured to digitize and wirelessly transmit the UA and TAE signals to the patient monitor 20. Alternatively or additionally, the fHR monitor may include an AFE or other digitization circuitry and a wireless transceiver and configured to communicate the fHR (and/or the entire maternal heart signal) to the patient monitor 20.

Alternatively, the set of electrodes 39 may be configured with a flexible connection cable 31 configured to removably connected to the patient monitor via connectors (such as connection ends 54 and 56 described in FIG. 2). Flexible cable 31 is configured to transmit the analog UA signal and the TAE signals obtained via the set of electrodes 39 to the patient monitor 20. In some embodiments, the set of electrodes 39 is positioned on the upper portion of the maternal patient's abdomen, near the fundus 43 of the maternal abdomen. In some embodiments, the flexible cable 31 is configured to allow placement of the patient monitor (or other receiving device) at various locations on or near the patient. Flexible cable 31 is configured to allow various relative positioning of the set of electrodes 39 and the patient monitor 20, and also to allow each element to be moved on the maternal abdomen without disturbing the other. For example, the flexible cable 31 may have a length and flexible construction configured to allow such relative movement. In one embodiment, the flexible cord 31 has a length of at least 15 cm or greater, and in another example may have a length of up to 2 feet. Some embodiments may be differently configured. An embodiment of a flexible cord 31 may be shorter than 15 cm and/or a flexible cord may be more than 2 feet, for example up to 5 feet or more. Additionally or alternatively, at least one flexible cord 31 may be configured as an active cable that includes sensors and/or parts of the signal processing circuitry for processing the signals obtained from the maternal patient.

In some embodiments, the controller 18 may include a central processing unit (CPU) and integrated memory. In the illustrated embodiment, the fetal monitoring system 10 includes a computer-readable medium (CRM) 14 that is communicatively connected to the controller 18 within the patient monitor 20. The controller exemplarily includes a processor that accesses software or firmware in the form of computer-readable code stored on non-transient computer-readable memory as either integrated memory or external memory. The processor executes the computer-readable code as an instruction set to carry out the functions as described herein, including the receipt of input, calculations, and outputs as will be described.

The patient monitor 20 includes a power source 12. The power source 12 may be a battery, such as a rechargeable battery. Alternatively or additionally, the power source 12 may be configured to be connected to an AC power source, such as to be plugged into a wall outlet providing grid power, and thus may include a DC converter and/or other power control circuitry.

Also included as part of the patient monitor 20 is a user interface 24. User interface 24 may be a display screen, a speaker, an LED light bulb, or any other type of interface that can generate an alert to a caregiver who is monitoring a maternal and fetal patient. When controller 18 detects a change in fHR such as commensurate with an indication of fetal distress an alert can be generated through user interface 24 to alert a caregiver of such a condition. In some embodiments, when the controller 18 detects a heartbeat coincidence between the maternal patient and the fetal patient, an alert can be generated to alert a caregiver to reposition the fHR monitor 66 to ensure that the fetal heart rate monitor 66 is picking up the fetal heart rate and not the maternal heart rate.

FIG. 2 is another schematic diagram of an exemplary embodiment of a maternal and fetal monitoring system 10 comprising a set of electrodes 39 configured to record a UA signal from the abdomen of a maternal patient, wherein at least one of the electrodes 39a-39c of the electrode set 39 is configured as a low pass filter to obtain the UA signal. In the illustrated embodiment, the electrode set 39 comprises a plurality of electrodes 39a-39c, including one electrode 39a configured to only pass signals below a threshold frequency to obtain the UA signal. A second electrode 39b and a third electrode 39c, at least one of which may be smaller than the first electrode 39a, are configured to obtain the TAE signal. Numerous other embodiments for the set of electrodes 39 are described herein. Data and/or signals acquired by the set of electrodes 39, the ultrasound transducer 28, the controller 18, and/or any other components in the housing 26 are communicated to a separate patient monitor 20 via a wired connection and/or wireless communications. In the illustrated embodiment, the first electrode 39a and the second electrode 39b are depicted as being generally the same size as one another. As such, the first electrode 39a and the second electrode 39b of FIG. 2 are both configured such that they may be used by the system 10 as a low-pass electrode. Some embodiments, however, may be differently configured. For example, some embodiments of an electrode set 39 may include one large electrode *(e.g.,* the first electrode 39a of FIG. 2) for obtaining the UA signal and a pair of smaller electrodes (e.g., the third electrode 39c of FIG. 2) for obtaining the TAE signal.

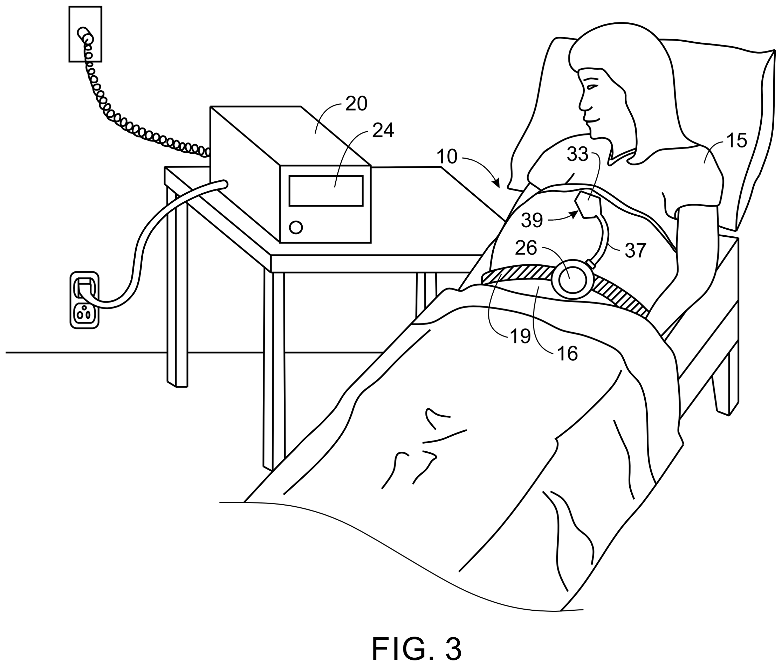

In the embodiments of FIG. 2, the set of electrodes 39 is connected to a housing 26 that is configured to be positioned on the abdomen of a maternal patient and comprises an ultrasound transducer 28 secured on a maternal patient abdomen to acquire fetal ultrasound measurements of a fetal patient. In operation, the ultrasound crystals serve as both ultrasound transmitters and ultrasound receivers and the ultrasound transducer 28 is operated to produce the acoustic waveform as described and then operated in a receive mode to receive the returned reflected acoustic signals back at the ultrasound transducer 28. The acoustic signals produced by the ultrasound transducer 28 reflect off of the anatomical structures of the fetal patient, particularly the fetal heart. The movement of the fetal heart causes the Doppler shift, which is detected and thus the fHR is measured. As discussed in further detail below, the ultrasound transducer 28 may be exemplarily secured to the abdomen of a maternal patient for example by way of an elastomeric strap 19 (FIG. 3). However, it will be recognized that in other embodiments the housing 26 may be maintained against the skin of the maternal abdomen by other means, such as by a biocompatible adhesive.

With continued reference to FIG. 2, the set of electrodes 39 are configured to be secured on a maternal patient abdomen to obtain physiological measurements indicative of UA of the maternal patient and the TAE signal, as described herein. A controller 38 located within first housing 26 uses the UA signal of the maternal patient and the TAE signal to determine the mHR, and in some embodiments also the fHR. The first housing 26 further comprises a power source 42. The power source 42 may be configured as a battery, such as a rechargeable battery. Some embodiments, however, may be differently configured.

In the embodiment of FIG. 2, the set of electrodes 39 is removably connected to first housing 26 through at least one flexible connection cable 31 configured to removably connected to first housing 26 via connection end 56 and connection port 54. Connection end 56 and connection port 54 are configured to matably connect in order to transmit the UA signals and the TAE signals from the set of electrodes to the controller 38. In some embodiments, connection end 56 and connection port 54 are male and female connectors, respectively. In other embodiments, connection end 56 and connection port 54 are female and male connectors, respectively. Additionally or alternatively, in some embodiments, the connection end 56 and connection port 54 may be configured to transmit power from the first housing 26, such as from the battery or other power source 42, to any powered devices on the measurement patch *(e.g.,* the ultrasound transducer 28, the electrodes 39a-39c, etc.).

In some embodiments, the controller 38 may include a central processing unit (CPU) and integrated memory. In the illustrated embodiments, the fetal monitoring system 10 includes a computer readable medium (CRM) 40 that is communicatively connected to the controller 38 within the housing 26. The controller 38 exemplarily includes a processor that accesses software or firmware in the form of computer-readable code stored on non-transient computer-readable memory as either integrated memory or external memory. The processor executes the computer-readable code as an instruction set to carry out the functions as described herein, including the receipt of input, calculations, and outputs as will be described.

With continued reference to FIG. 2, the controller 38 is configured to transmit the UA signal, as well as the TAE signal and/or the total heart (TH) signal to the patient monitor 20, which may be by wired or wireless means. In the depicted embodiment, the housing 26 includes transceiver 60 configured to transmit the signals to the patient monitor 20. Alternatively or additionally, the controller 38 may be configured to identify the maternal heart signal component of the TH signal and/or determine the mHR by removing the fetal heart signals (determined via the doppler ultrasound transceiver 28) from the TH signal. In such an embodiment, the controller 38 is further configured to transmit the maternal heart signal and/or the mHR to the patient monitor 20. The transceiver 60 may be any device known in the art for wirelessly transmitting data between two points. In one embodiment, the transceiver 60 may be a body area network (BAN) device, such as a medical body area network (MBAN) device, that operates as part of a wireless network of wearable or portable computing devices. Other examples of radio protocols that could be used for this purpose are Bluetooth, Bluetooth Low Energy (BLE), ANT and ZigBee. The external patient monitor may exemplarily display the physiological data for a caregiver's attention or may store the data electronically, such as in an electronic patient record.

Also included within the first housing 26 is a user interface 58. The user interface 58 may be a display screen, a speaker, an LED light bulb, or any other type of interface which can generate an alert to a caregiver who is monitoring a maternal and fetal patient. In some embodiments, when controller 38 detects a drop in fetal heart rate such as is commensurate with an indication of fetal distress, an alert can be generated through user interface 58 to alert a caregiver of such a condition. Additionally or alternatively, when the controller 38 detects a heartbeat coincidence between the maternal patient and the fetal patient an alert can be generated to alert a caregiver to reposition the ultrasound transducer 28 to ensure ultrasound transducer 28 is picking up the fetal heart rate and not the maternal heart rate.

A flexible cable 31 is configured to transmit the UA signal and the TAE signal to first housing 26. In some embodiments, the set of electrodes 39 is configured to be positioned on the upper portion of the maternal patient's abdomen, near the fundus of the uterus. In some embodiments, the flexible cable 31 is configured to allow placement of the first housing 26 on the lower portion of the maternal patient's abdomen while the set of electrodes 39 is positioned at the fundus 43. Thus, the first housing 26 may be positioned level with or below the umbilicus, near the location of the fetal patient, to detect and track fHR. Flexible cable 31 is configured to allow various relative positioning of the set of electrodes 39 and the first housing 26, and also to allow each element to be moved on the maternal abdomen without disturbing the other. For example, the flexible cable 31 may have a length and flexible construction configured to allow such relative movement. In one embodiment, the flexible cord 31 has a length of at least 15 cm or greater, and in another example may have a length of up to 2 feet. Some embodiments may be differently configured. An embodiment of a flexible cord 31 may be shorter than 15 cm and/or a flexible cord may be more than 2 feet, for example up to 5 feet or more. Additionally or alternatively, at least one flexible cord 31 may be configured as an active cable that includes sensors and/or parts of the signal processing circuitry for processing the signals obtained from the maternal patient.

FIG. 3 is an environmental view of an exemplary embodiment of a maternal and fetal monitoring system 10 that can be used to simultaneously monitor the fetal patient's heart rate via doppler ultrasound and to utilize a plurality of electrodes to measure the maternal patient's uterine activity and the TAE signal, from which the maternal heart rate can be determined. The maternal and fetal monitoring system 10 is exemplarily secured to the abdomen 16 of the maternal patient 15 for example by way of an elastomeric strap 19 attaching the ultrasound transducer and a biocompatible adhesive is used to secure the set of electrodes 39 to the upper portion of the patient's abdomen. It will be recognized that in other embodiments a biocompatible adhesive may also be used to secure the housing 26 of the ultrasound transducer to the patient's abdomen 16 instead of the strap 19. In the embodiment of FIG. 3, the set of electrodes 39 are shown mounted to a single patch 33, various embodiments of which are described herein. The electrode set 39 is communicatively connected to the housing 26 via a flexible electrical connector 37. The flexible electrical connector 37 may include one or more electrical lines, or leadwires, that connect each of the electrodes 39a-39c to the housing 26. For example, the electrical connector 37 may include ringed circuit components for summing the signals and/or for providing a low-pass filtering effect on the signal.

In the depicted embodiment, the electrode set 39 is mounted on a single patch (or other substrate) such that the first electrode 39a, second electrode 39b, and third electrode 39c are in fixed locations relative to each other. In other embodiments, electrode set 39 may be configured such that one or more of the electrodes is movable with respect to the others, such as wherein the second electrode 39b and/or the third electrode 39c are movable relative to the first electrode 39a. In such an embodiment, the second electrode 39b and/or the third electrode 39c may be mounted on a separate substrate from the first electrode 39a. In some embodiments, each electrode may be flexibly connected to the first electrode 39a, such as by a serpentine-shaped printed leadwire on a serpentine-shaped flexible substrate (such as a foam substrate) that can be adjusted to adjust the location and distance between the various parts of the patch. For example, the distance between the electrodes 39a-39c may be as low as 1cm or as high as 20 cm.

The maternal and fetal monitoring system 10 may be communicatively connected to an external patient monitor 20. As will be understood by the variety of implementations as described in further detail herein, while all remaining within the scope of the present disclosure, the communicative connection may exemplarily be a wired or a wireless communicative connection. A wireless communicative connection may be a medical body area network (MBAN), and/or may exemplarily use Bluetooth, Bluetooth Low Energy (BLE), ANT or ZigBee communication protocols or other RF communication protocols as may be recognized by a person of ordinary skill in the art. In still further exemplary embodiments, depending upon the configuration of the maternal and fetal monitoring system and the data transmitted between the maternal and fetal monitoring system 10 and the external patient monitor 20, all or some of the data processing of the physiological information acquired by the maternal and fetal monitoring system 10 may be performed locally by a controller within the maternal and fetal monitoring system 10, such as by a controller 38 located within first housing 26. The calculated parameters of fetal heart rate, maternal heart rate, uterine activity, or others as described herein may be communicated across the communicative connection to the external patient monitor 20 exemplarily for visual presentation on a graphical display and/or electronic storage of this information on a data network of the hospital or medical facility and exemplarily in an electronic medical record (EMR) of the maternal patient.

Additionally or alternatively, a controller located within the external patient monitor 20 may receive some or all of the acquired physiological data and process such physiological data in the manners as described herein. In these embodiments, the maternal and fetal monitoring system 10 may perform more limited signal processing on the acquired physiological data and provide this "raw" physiological data across the communicative connection to an external patient monitor 20 which applies the signal processing actions and techniques as described herein to calculate the parameters of fetal heart rate, maternal heart rate, and others.

As previously mentioned, an embodiment of a maternal and fetal monitoring system 10, such as the systems 10 of FIGS. 1-3, may be configured to obtain physiological measurements indicative of UA of the maternal patient and the TAE signal, and to use said physiological measurements to calculate and/or provide a user with UA data, fHR data, and mHR data from the maternal patient 15. For example, using the set of electrodes 39 and a fHR monitor 66 (*e.g.,* an ultrasound transducer 28), embodiments of a maternal and fetal monitoring system 10 may be configured to obtain the TAE and UA signals. The TAE and UA signals may then be used to calculate a TH signal, and the TH signal and the fetal heart signals from the fHR monitor may be used to calculate the mHR.

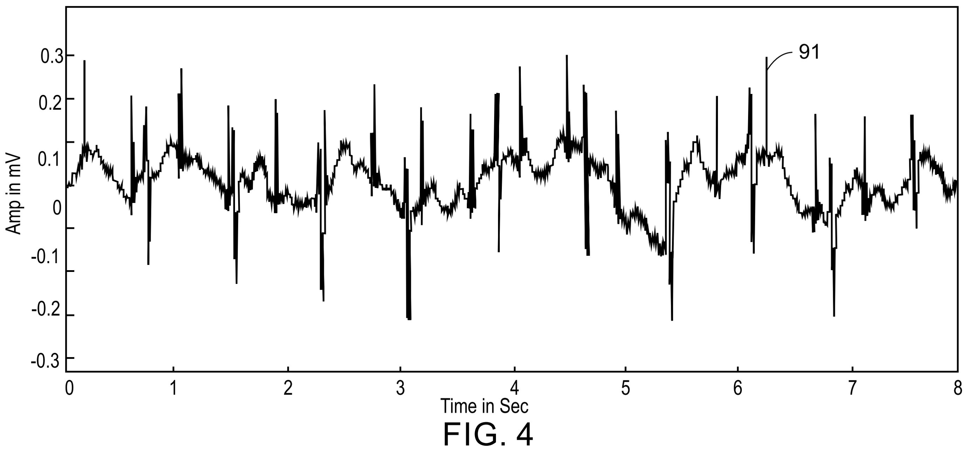

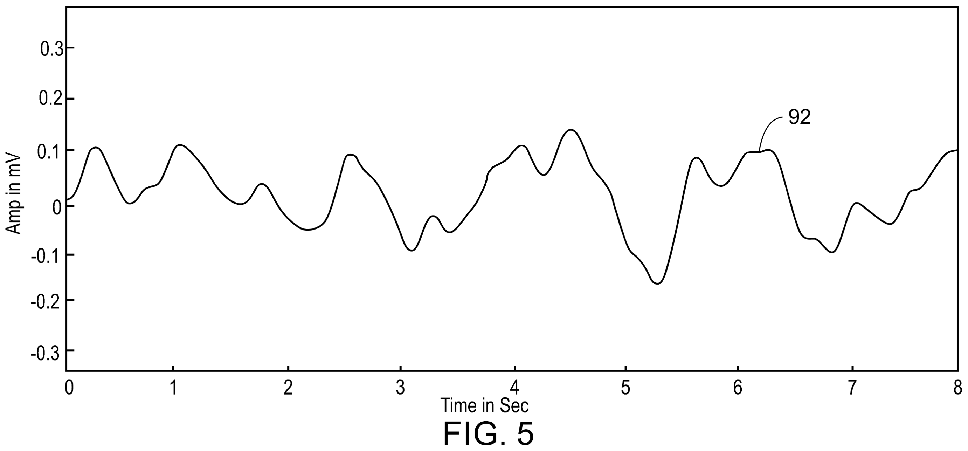

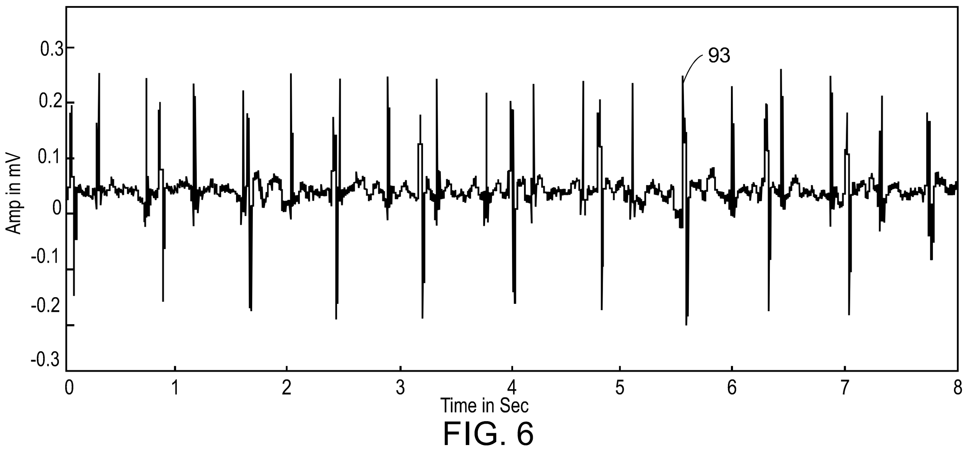

FIG. 4 is a graph showing an exemplary total abdominal electrical (TAE) signal 91 obtained via the set of electrodes, and FIG. 5 is a graph showing an exemplary uterine activity (UA) signal 92 obtained by the same set of electrodes. Various embodiments and configurations of the set of electrodes are described herein, wherein the set of electrodes is configured to act as a low pass filter to obtain the UA signal. FIG. 6 shows the total heart (TH) signal 93 calculated by removing the UA signal from the TAE signal.

For example, embodiments of a maternal and fetal monitoring system 10 (*i.e.,* the system 10 of FIGS. 2 and 3) include a set of electrodes 39 configured to be positioned on the abdomen 16 of a maternal patient 15 (FIG. 3) and obtain the TAE signal, which includes the UA signal, the maternal heart signal, and the fetal heart signal. Advantageously, the set of electrodes 39 includes at least one electrode 39a configured to act as a low pass filter to obtain the UA signal without requiring any signal processing hardware. Using the set of electrodes 39 and an ultrasound transceiver 28 (or another fHR monitor 66 device) positioned on the maternal abdomen 16, the maternal and fetal monitoring system 10 can be used to monitor UA signals, fetal heart signals, and maternal heart signals.

Referring to FIGS. 1-6, as previously mentioned, the maternal and fetal monitoring system 10 references at least one electrode configured as a low pass filer to acquire the UA signal from the maternal abdomen 16. In the system 10 of FIGS. 1 and 2, the first electrode 39a is a low pass electrode 39a that is configured in a monopolar arrangement for obtaining the UA signal. In some embodiments, the system 10 may be configured to reference a reference electrode (not shown) in order to obtain the UA signal with the monopolar, low-pass electrode 39a.. The reference electrode may be placed at a location other than the abdomen 16 on the maternal patient (e.g., a right leg electrode, a right arm electrode, or an electrode placed on the patient's back). The second electrode 39b and the third electrode 39c, which are not included in the subset for obtaining the low frequency signal, are configured to be used in a bipolar arrangement to obtain the TAE signal from the maternal patient 15. The signal second electrode 39b is referenced to the third electrode 39c and/or another electrode, which may be the first electrode 39a, the reference electrode, or may be another electrode placed elsewhere on the maternal patient (e.g., a left or right leg electrode, a left or right arm electrode, or an electrode placed on the patient's back). In some embodiments, a maternal and fetal monitoring system 10 may be configured to use the second electrode 39b or the third electrode 39c in a monopolar arrangement with reference to a reference electrode (not shown) in order to obtain the TAE signal.

In some embodiments, the filtering characteristics of the low pass electrode 39a may be based on the dimensions of the low pass electrode 39a and/or the materials with which the low pass electrode 39ais constructed. For example, the filtering characteristics of a low pass electrode 39a may be a function of the width of the electrode 39a, the height of the electrode 39a, the thickness of the electrode 39a, the material(s) with which the electrode 39a is constructed, the placement and orientation of the low pass electrode 39a on the maternal abdomen (*e.g*., the orientation of the electrode 39a relative to the muscle fibers in the tissue below the electrode 39a), and/or the dimensions and materials of any additional components on the low pass electrode patch. Similarly, the material(s), dimensions (*e.g.,* width, height, thickness), position, and/or orientations of the second electrode 39b and third electrode 39c may be selected to achieve the desired characteristics for the second electrode 39b and third electrode 39c.

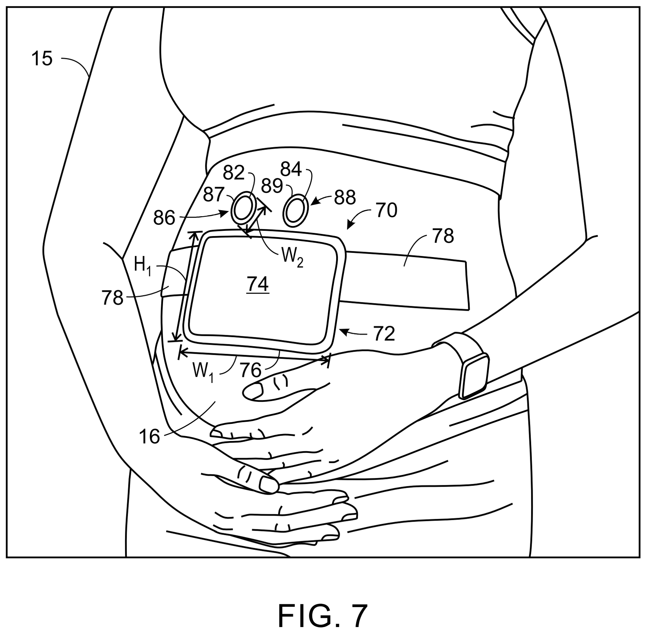

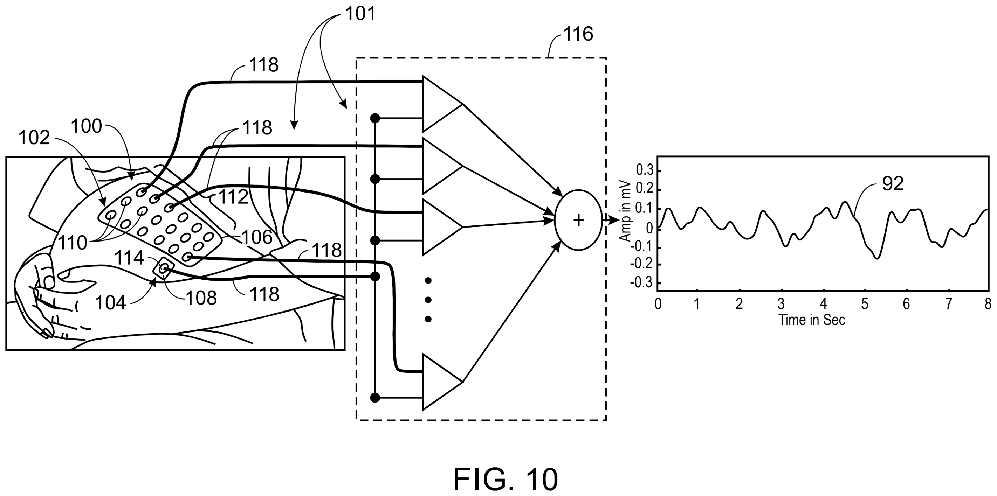

The maternal and fetal monitoring system 10 may be configured to adaptively select the first electrode 39a, the second electrode 39b, or some subset of the electrodes in the electrode set 39 (or any of the electrode set 100, 120, 140, 160, 180 embodiments described in FIGS. 10-14), to be used to acquire the UA signal 92. For example, the system 10 may adaptively select either the first electrode 39a or the second electrode 39b to be used in a monopolar configuration in reference to a reference electrode (not shown) in order to obtain the UA signal. Some embodiments of the system 10, however, may be configured to adaptively select a subset of low pass electrodes (*e.g.,* the first and second electrodes 39a, 39b) to be used in a bipolar arrangement to obtain the UA signal. Alternatively, a maternal and fetal monitoring system 10 may use a predetermined electrode in a monopolar configuration or a predetermined subset of electrodes in a bipolar configuration, such as electrodes 39a and 39b having a larger electrode dimension configured to pass only low frequencies, for acquiring the UA signals. In some embodiments, the electrode set may include just one larger electrode configured to pass only low frequences, such as the embodiments shown in FIGS. 7-9. In such an embodiment, the system 10 (such as via the controller 18, 38) may be configured to select the UA signal 92 from the bipolar output between the large electrode 74 and either of the two smaller electrodes 82 and 84. In still other embodiments, the UA signal may be obtained by utilizing a linear array of electrodes or a grid of electrodes and combining (*e.g*., by summing or averaging) the outputs therefrom, such as by the inclusion of logic gates or other hardware elements configured to sum the signals from multiple electrode sets that are placed closely together. Where multiple electrode pairs are configured to provide the low pass filtering effect, the electrode pair (or pairs) used for generating the UA signal 92 may be selected, for example, based on the signal strength of the acquired UA signal and/or the signal-to-noise ratio.

Similarly, the electrode(s) utilized for obtaining the TAE signal 91 (e.g., FIG. 4) are obtained from the maternal abdomen 16, and the system 10 may be configured to obtain the TAE signal 91 from an electrode in a monopolar configuration (e.g., the second electrode 39b or third electrode 39c), which may be predetermined or adaptively selected, by referencing a predetermined reference electrode (e.g., one of the other electrodes in the electrode set or a designated reference electrode located elsewhere on the patient). Some embodiments, however, may be configured to us a predetermined or adaptively selected set of electrodes (e.g., the second and third electrodes 39b, 39c) in a bipolar configuration to obtain the TAE signal. The maternal and fetal monitoring system 10 may then generate a TH signal 93 (e.g., FIG. 6) by subtracting the UA signal from the TAE signal. The resulting TH signal contains at least the maternal heart signal and the fetal heart signal.

The maternal and fetal monitoring system 10 may be configured to execute subtraction of the UA signal from the TAE signal to generate the TH signal. This may be performed via digital signal processing means via the processor. Alternatively, the signal subtraction may be executed using various hardware elements and/or logic gates. For example, the maternal and fetal monitoring system 10 may comprise hardware amplifiers (not shown) and/or other circuit elements for subtracting the analog UA signal 92 from the analog TAE signal 91 to generate a third output signal to generate the TH signal as a third analog input signal to the system. In various embodiments, such hardware may be located on or adjacent to the patch hosting the electrodes (e.g., patch 33), in the flexible electrical connector 31, 37 (or another electrical line), or in the housing of the patient monitor or intermediary device (e.g., within housing 26). As previously mentioned, in some embodiments, the UA signal may be removed from the TAE using other linear and/or non-linear processing methods.

After the TH signal has been generated, the maternal and fetal monitoring system 10 may then determine the mHR from the TH signal using the fetal heart signals and/or fHR, such as the fHR that is separately obtained by the ultrasound transducer 28 (or another fHR monitor 66 device). The UA signal, the mHR signal, and/or the fHR signal may then be output from the system 10 to the patient monitor 20 and/or another external device. A user interface 24 on the illustrated patient monitor may be configured to display the determined UA, mHR signal, and/or the fHR signals. Advantageously, placement of the set of electrodes 39 on the maternal abdomen 16 proximate the fundus region may result in an fHR signal with a relatively low magnitude compared to the magnitude of the mHR signal. This may be useful so that the mHR signal can be obtained from the TH signal without removing the fHR signal from the TH signal.

Embodiments of a set of electrodes for a maternal and fetal monitoring system 10 may include electrodes in a plurality of different arrangements. FIG. 7 illustrates another exemplary embodiment of a set of electrodes 70 configured to acquire and communicate signals from the maternal patient 15 to an external patient monitor device via a wired and/or wireless connection. The illustrated set of electrodes 70 includes a first electrode patch 72 with a low pass electrode 74 and relatively smaller second and third electrode patches 86, 88 that respectively include second and third electrodes 82, 84. Each of the electrodes 74, 82, 84 is mounted on a substrate 76, 87, 89 configured to adhere to the abdomen 16 of the maternal patient 15. The low pass electrode 74 is mounted on a first substrate 76 and, in some embodiments, may include at least one strap 78 that extends outward from the first patch substrate 76. The second electrode 82 and the third electrode 84 are respectively mounted on a second substrate 87 and a third substrate 89. In some embodiments, a skin-safe adhesive may be disposed on at least a portion of the first electrode patch 72, the second electrode patch 86, and/or the third electrode patch 88 to secure said electrode patch 72, 86, 88 to the maternal abdomen 16.

With continued reference to FIG. 7, the low pass electrode 74 is dimensioned such that it is only configured to pass (*i.e.,* detect) signals that have a frequency that falls below a threshold frequency. For example, the low pass electrode 74 of FIG. 7 is dimensioned with a width W₁ and a height H₁ that are significantly larger than the diameters W₂ of the second electrode 82 and third electrode 84 such that the contact area between the low pass electrode 74 and the abdomen 16 is larger than the contact areas between the second and third electrodes 82, 84 and the abdomen 16. The dimensions (*i.e.,* width W₁, height H₁, and/or thickness) of the low pass electrode 74 determine the value of the threshold frequency for the low pass electrodes 74. As such, the low pass electrode 74 can be dimensioned so that it only passes low frequency signals associated with UA signal. In the illustrated embodiments, the width W₁ of the low pass electrode 74 may be at least 10 centimeters and the diameter W₂ of the second and third electrodes 82, 84 may be 2 centimeters or smaller. Other embodiments may be differently configured. For example, a maternal and fetal monitoring system 10 may include at least one low pass electrode 74 with a width W₁ of at least 15 centimeters. In some embodiments, a low pass electrode 74 may be configured with a width W₁ of at least 20 centimeters. In some embodiments, the thickness of a low pass electrode 74 may be 0.1 cm to 0.5 cm thick. some embodiments may have at least one electrode that is less than 0.1 cm thick and/or at least one electrode that is more than 0.5 cm thick.

In some embodiments, at least one of the second and third electrodes 82, 84 may be configured with a width W₂ of 2 centimeters or less. In some embodiments, the electrode 74 (or subset if electrodes 74, 82, 84) of the electrode set 70 configured to act as the low pass filter may be configured to only pass frequencies below 2 Hz. Some embodiments, however, may be differently configured. For example, embodiments of a low pass electrode 74 may be configured to only pass frequencies below a different threshold value (*e.g.*, 1 Hz, 0.5 Hz, 0.1 Hz, etc.).

In some embodiments, the filtering characteristics of the low pass electrode(s) 74 may be based on the materials with which the low pass electrode patch 72 is constructed. For example, in addition to being a function of its width W₁ and height H₁, the threshold frequency of a low pass electrode 74 may be a function of the material(s) with which the electrode 74 is constructed, the thickness of the electrode 74, the placement and orientation of the low pass electrode 74 on the maternal abdomen (*e.g*., the orientation of the electrode 74 relative to the muscle fibers in the tissue beneath said electrode 74), and/or the dimensions and materials of any additional components on the low pass electrode patch 72. For example, in some embodiments, increasing the thickness of the low pass electrode 74 may increase the low pas filtering effect of the low pass electrode 74. Other embodiments may include a low pass electrode 74 formed from a material such that increasing the thickness of the low pass electrode 74 may increase the value of the frequency threshold of the low pass electrode 74. In some embodiments, the material and/or dimensions (width W₂, height, thickness) of at least one of the second and third electrodes 82, 84 may be selected to achieve the desired characteristics for the non-low pass electrodes 82, 84.

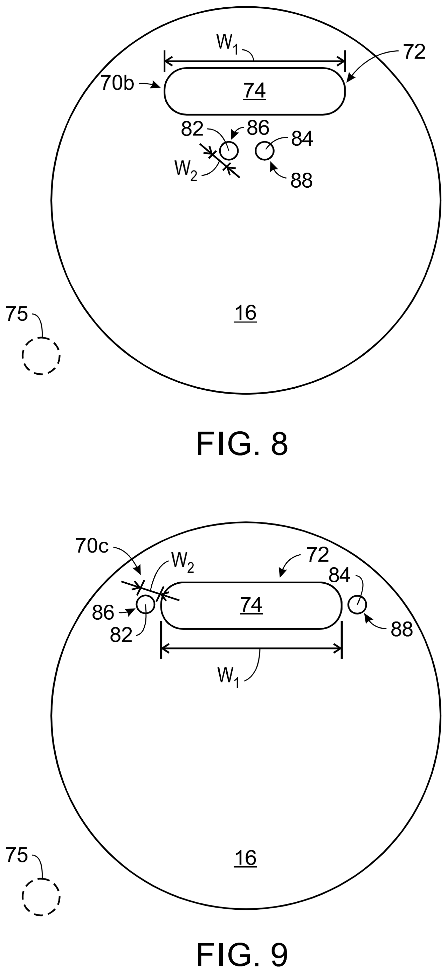

In the embodiment of FIGS. 7-9, the electrode set 70 includes a single low-pass electrode 74, which in these embodiments is a large electrode, and two additional smaller electrodes 82, 84. To obtain the UA signal, the maternal and fetal monitoring system 10 may be configured to use the low-pass electrode 74 in a monopolar configuration with reference to a reference electrode 75 (shown schematically in FIGS. 8 and 9). The reference electrode 75 may be placed at a location other than the abdomen 16 on the maternal patient (e.g., a right leg electrode or an electrode placed on the patient's back). Alternatively, some embodiments of a maternal and fetal monitoring system 10 may be configured to adaptively select the second electrode 82 and/or the third electrode 84 (or some subset of electrodes) to be used in a bipolar arrangement with the low pass electrode 74. In such an embodiment, the system 10 (such as via the controller 18, 38) may be configured to select the UA signal 92 from the bipolar output between the large electrode 74 and either of the two smaller electrodes 82 and 84. Where multiple electrode pairs are configured to provide the low pass filtering effect, the electrode pair (or pairs) used for generating the UA signal 92 may be selected, for example, based on the signal strength of the acquired UA signal and/or the signal-to-noise ratio. Some embodiments, however, may be differently configured. For example, a maternal and fetal monitoring system 10 may use a predetermined subset of electrodes for acquiring the UA signals and a separate electrode (or set of electrodes) for acquiring the TAE signal.

Similarly, in the embodiment of FIGS. 7-9, the electrode(s) utilized for obtaining the TAE signal 91 (FIG. 4) are obtained from the maternal abdomen 16, and the maternal and fetal monitoring system 10 may be configured to obtain the TAE signal 91 from a subset of electrodes (*e.g.,* the second and third electrodes 82, 84) in a bipolar configuration, or by using a single electrode (*e.g.,* the second or third electrode 82, 84) in a monopolar configuration by referencing a reference electrode (e.g., one of the other electrodes in the electrode set 100 or a designated reference 75 electrode located elsewhere on the patient). The electrode(s) 74, 82, 84 used to obtain the TAE signal may be predetermined or adaptively selected by the system 10. The maternal and fetal monitoring system may then generate a TH signal 93 (FIG. 6) by removing the UA signal from the TAE signal, for example by subtraction. The resulting TH signal contains at least the maternal heart signal and the fetal heart signal.

In some embodiments, the removal of the UA signal from the TAE signal (via subtraction of the UA signal and/or other processing methods) may be performed via digital signal processing means via the processor. Alternatively, the signal removal of the UA signal from the TAE signal may be executed using various hardware elements and/or logic gates. For example, the maternal and fetal monitoring system may include hardware amplifiers (not shown) and/or other circuit elements for subtracting the analog UA signal 92 from the analog TAE signal 91 to generate a third output signal to generate the TH signal 93 as a third analog input signal to the system. In various embodiments, such hardware may be located on or adjacent to the patch hosting the electrodes (e.g., on or adjacent to the first, second, or third substrate 76, 87, 89), in the flexible electrical connector (e.g., flexible cable 31), or in the housing of the patient monitor or intermediary device (e.g., within housing 26). The mHR may be determined from the TH signal using the fetal heart signals and/or fHR, such as the fHR that is separately obtained by the ultrasound transducer 28 (or another fHR monitor 66 device). The UA signal, the mHR signal, and/or the fHR signal may then be output from the maternal and fetal monitoring system to the patient monitor 20 and/or another external device.

In the embodiment of FIG. 7, the second electrode patch 86 and the third electrode patch 88 are located above the first electrode patch 72 on the abdomen 16 of the maternal patient 15. This arrangement advantageously results in relatively small fetal heart signals that are easily filtered out, for example to obtain the mHR. Some embodiments, however, are differently configured. For example, the electrode patches 72, 86, 88 of FIG. 7 are each independently placeable on the maternal abdomen 16 such that the electrodes 74, 82, 84 may be positioned independently of one another, for example based on the signal strength of the electrode.

Referring to FIG. 8, an embodiment of an electrode set 70b for a maternal and fetal monitoring system 10 includes a first electrode patch 72 with a low pass electrode 74 and relatively smaller second and third electrode patches 86, 88 that include second and third electrodes 82, 84, respectively. Each of the electrodes 74, 82, 84 is mounted on a substrate 76, 87, 89 (FIG. 7) configured to adhere to the abdomen 16 of the maternal patient 15. The low pass electrode 74 is mounted on a first substrate 76 and the second electrode 82 and the third electrode 84 are respectively mounted on a second substrate 87 and a third substrate 89. In some embodiments, a skin-safe adhesive may be disposed on at least a portion of the first electrode patch 72, the second electrode patch 86, and/or the third electrode patch 88 to secure said electrode patch 72, 86, 88 to the maternal abdomen 16. In the embodiment of FIG. 8, at least one of the second electrode patch 86 and the third electrode patch 88 may be positioned below the low pass electrode patch 72 such that the low pass electrode 74 is positioned above the respective second and/or third electrodes 82, 84.

FIG. 9 shows an embodiment of an electrode set 70c for a maternal and fetal monitoring system 10 includes a first electrode patch 72 with a low pass electrode 74 and relatively smaller second and third electrode patches 86, 88 that respectively include second and third electrodes 82, 84. Each of the electrodes 74, 82, 84 is mounted on a substrate 76, 87, 89 (FIG. 7) configured to adhere to the abdomen 16 of the maternal patient 15. The low pass electrode 74 is mounted on a first substrate 76 and the second electrode 82 and the third electrode 84 are respectively mounted on a second substrate 87 and a third substrate 89. In some embodiments, a skin-safe adhesive may be disposed on at least a portion of the first electrode patch 72, the second electrode patch 86, and/or the third electrode patch 88 to secure said electrode patch 72, 86, 88 to the maternal abdomen 16. In the embodiment of FIG. 9, at least one of the second electrode patch 86 and the third electrode patch 88 may be positioned on a lateral side of the low pass electrode patch 72 such that the respective second and/or third electrodes 82, 84 are spaced laterally apart from the low pass electrode 74.

In some embodiments, the filtering characteristics of the low pass electrode 74 of FIGS. 8 and 9 may be based on the dimensions of the low pass electrodes 74 and/or the materials with which the low pass electrode 74 is constructed. For example, the filtering characteristics of a low pass electrode 74 may be a function of the width W₁ of the electrode 74, the height H₁ of the electrode 74, the material(s) with which the electrode 74 is constructed, the thickness of the electrode 74, and/or the dimensions and materials of any additional components on the low pass electrode patch.

The size, location, and orientation of each electrode 74, 82, 84 in the electrode set 70 may be different than those illustrated in FIGS. 7-9. For example, in some embodiments, the larger, low pass electrode 74 may have a width W₁ that is at least 15 centimeters (*e.g.,* 15 cm, 20 cm, 25 cm, etc.) and the smaller electrodes 82, 84 may have a width W₂ that is at least 0.5 centimeters and up to 2 centimeters (*e.g.,* 1 cm or 1.5 cm). In one embodiment, the smaller electrodes are round, and thus the diameter is at least 0.5 cm and not greater than 2 cm. Similarly, the spacing between each of the electrodes 70, 82, 84 may vary. For example, the second and/or third electrodes 82, 84 may positioned on the maternal abdomen 16 at least 1 cm separation distance from the low pass electrode 74, and not more than 20 centimeters apart from the low pass electrode 74.

In some embodiments, the set of electrodes may include an array of electrodes that are collectively used to obtain the UA signal, and in some embodiments also the TAE signal from the maternal patient 15. FIG. 10, for example, illustrates an embodiment of a maternal and fetal monitoring system 101 comprising an electrode set 100 that has a first electrode patch 102 including a plurality of individual electrodes 110 arranged in an array 112 and a second electrode patch 104 with one electrode 114. As discussed in further detail below the electrode array 112 is configured to act as a low pass filter for obtaining the UA signal. Each of the electrodes 110 in the electrode array 112 and the electrode 114 on the second electrode patch 104 are connected to an external device (not shown), for example a patient monitor, via at least one electrical connector 118 extending between the electrode patches 102, 104 and said external device. UA signals, heart signals, and/or heart rate information may be communicated to the external device via the electrical connector(s) 118. For example, processed or unprocessed UA and heart signals may be communicated, directly or indirectly, to a patient monitor device via at least one electrical connector 118. Additionally or alternatively, at least one electrode 110, 114 may be configured to wirelessly communicate data to an external device.

In the embodiment of FIG. 10, the electrode array 112 includes a plurality of electrodes 110 that are mounted on a substrate 106 of the first electrode patch 102. The electrode array 112 on the exemplary first electrode patch 102 includes a total of eighteen electrodes 81 that are arranged in grid with three rows (linear arrays) of six electrodes 110 each. This arrangement is merely exemplary. In other embodiments, each linear array in the grid may be at least ten electrodes across, and in some embodiments fifteen or twenty electrodes across. By appropriately spacing the individual electrodes 110 on the first electrode patch 102 across the maternal abdomen 16, the outputs of electrodes in the electrode array 112 can be combined (*e.g.*, by adding, averaging, or otherwise combining the electrode outputs) and thus are configured to act as a low pass filter. In such an embodiment, the electrode array 112 is arranged in a monopolar arrangement with the electrode 114 used as the reference electrode, which in the depicted example is on separate electrode patch 104 from the larger patch 102 comprising the grid of electrodes and configured to be placed adjacent to the larger patch 102 when acquiring the UA signals. In some embodiments, the electrode array 112 may be configured in a monopolar arrangement and the UA signal may be obtained by referencing a reference electrode located elsewhere on the maternal patient's body (*e.g.,* on the maternal arm, leg, back, etc.). To obtain the TAE signal, the maternal and fetal monitoring system 101 may be configured to use at least one electrode 110 in the electrode array 112 and/or the electrode 114 on the second electrode patch 104. For example, the system 101 may be configured to use a pair of any two electrodes 110, 114 in a bipolar arrangement to obtain the TAE signal. Additionally or alternatively, some embodiments of the system 101 may be configured to use one of the electrodes 110 in the electrode array 112 or the electrode 114 on the second electrode patch 104 in a monopolar configuration to obtain the TAE signal from the maternal abdomen 16 by referencing a reference electrode on the maternal patient. In other embodiments, the electrode 114 may be mounted on the same patch as the electrode array 112 such that its relative location compared to the electrode array 112 is fixed. In still other embodiments, the outputs from the electrode array 112 may be generated by referencing pairs of electrodes 110 within the electrode array together. In some implementations, multiple electrodes 110 in the electrode array 112 may all be referenced to the same electrode. In other embodiments, the outputs may come from different combinations of electrode 110 pairs.

Hardware elements 116, such as integrated circuits, are provided to generate the output signals from the electrode array 112. For example, inputs from electrode pairs (such as each electrode 110 in the electrode array 112 paired with electrode 114, as shown) are provided as differential inputs to operational amplifiers. In other embodiments, different comparator and/or amplifier IC components may be utilized. For example, at least one hardware element 116 may be a printed circuit element that is disposed on the electrode patch 102, 104 or may be integrated into or a connector 118.

The outputs of the electrodes are then combined, for example by adding, averaging, or otherwise combining the outputs from the electrode) to obtain the UA signal 92. Thus, the hardware elements 116 include logic gates or other elements, such as IC elements hardware elements configured to sum the outputs from each of the multiple electrodes/ electrode pairs. Such hardware may be located on or adjacent to the patch 102 hosting the electrodes 110, in the flexible electrical connector 118 (or another electrical line), or in the housing of the patient monitor or intermediary device. In some embodiments, the maternal and fetal monitoring system 101 may be configured to adaptively select a subset of electrodes 110 (or pairs thereof) in the electrode array to be used to acquire the UA signal 92, for example, based on the signal strength of the acquired UA signal and/or the signal to noise ratio. Alternatively, some embodiments of a maternal and fetal monitoring system 101 may use a predetermined subset of electrodes for acquiring the UA signal. In other embodiments, the signals may be combined using signal processing software and techniques.

The TAE signal 91 (FIG. 4) may be obtained from the maternal abdomen 16 using the electrode 114, or from any single electrode or electrode pair in the electrode array 112. The maternal and fetal monitoring system 101 may then generate a TH signal 93 (FIG. 6) by removing the UA signal from the TAE signal. The resulting TH signal contains at least the maternal heart signal and the fetal heart signal.

After the TH signal has been generated, the maternal and fetal monitoring system 101 may then determine the mHR from the TH signal using the fetal heart signals and/or fHR that is separately obtained by an ultrasound transducer (or another fHR monitor device). The UA signal and the mHR signal may then be output from the system 101 to the patient monitor 20 and/or another external device. A user interface 24 on the illustrated patient monitor may be configured to display the UA and mHR signal.

As is described herein, the filtering characteristics of the low pass electrode patch 102 of FIG. 10 may be based on the dimensions of the electrodes 110 in the electrode array 112 and/or the materials with which the electrodes 110 are constructed. For example, the filtering characteristics of a low pass electrode patch 102 may be a function of the number of electrodes 110 in the electrode array 112, the spacing between the electrodes 110, the width of the electrode array 112, the height of the electrode array 112, the material(s) with which the electrodes 110 are constructed, the thickness of the electrodes 110, and/or the dimensions and materials of any additional components on the low pass electrode patch.

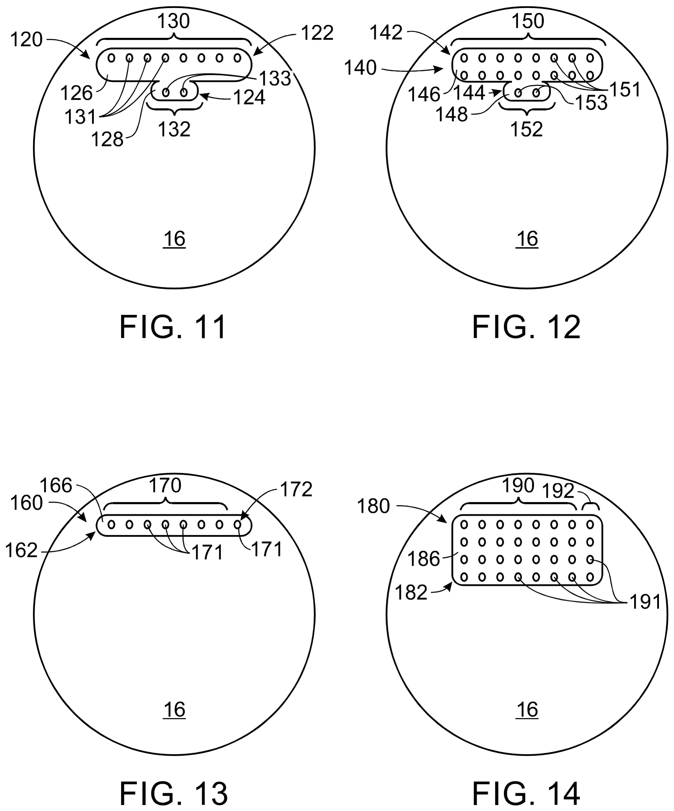

FIGS. 11-14 show different embodiments of electrode arrays that may be utilized to generate the UA signal according to the present disclosure. FIG. 11 illustrates an embodiment of an electrode set 120 including a first electrode array 130 on a first patch portion 122 and a second electrode array 132 on a second patch portion 124. The first electrode array 130 includes a linear arrangement of eight electrodes 131 mounted on a first substrate 126 of the first patch portion 122. The eight electrodes 131 are spaced laterally across the first patch portion 122 so that the first electrode array 130 may act as a low pass filter for acquiring the UA signal. The second electrode array 132 includes two electrodes 133 arranged linearly and mounted on a second substrate 128 of the second patch electrode 86.

The electrode set 120 may be connected to patient monitor or other external device and can be configured for monitoring UA activity and mHR of a patient 15. The electrodes 131 in the first electrode array 130 are configured to be used in a monopolar arrangement with a reference electrode (not shown) to function as a low-pass electrode. The output signals from the electrode(s)of the electrode array 130 (or some subset thereof) are combined (e.g., by adding together or averaging the electrode outputs) via hardware elements or software as described above to generate the UA signal.

At least one of the electrodes, such as electrode 133 in the second electrode array 132, may be used to obtain the TAE signal from the patient 15. The electrode(s) 131, 133 used to obtain the TAE signal may be used in a monopolar configuration by referencing a reference electrode, or as a pair of electrodes 131, 133 in a bipolar configuration. In some embodiments, the TAE signal may be acquired by referencing the electrodes 133 in the second electrode array 132 to obtain the TAE signal. The TH signal may then be generated by subtracting the UA signal from the TAE signal, as is described above.

In some embodiments, the size, orientation, and/or arrangement of the individual electrodes 131, 133 in an array of electrodes 130, 132 may differ from those of FIG. 11. For example, the individual electrodes 131, 133 in each of the illustrated electrode arrays 130, 132 may have a width that is between 0.2 cm and 0.5 cm. Some embodiments, however, may include at least one electrode that is larger than 0.5 cm and/or at least one electrode that is smaller than 0.2 cm. In some embodiments, the spacing between the electrodes 131, 133 of the first or second electrode arrays 130, 132 may be spaced between 0.4 cm and 1.5 cm apart from each other. For example, the spacing between the individual electrodes 131, 133 may be at least 0.8 cm and no more than 1 cm apart. Some embodiments, however, may include at least one electrode that is more than 1 cm apart, or more than 1.5 cm apart, from another electrode and/or at least one electrode that is less than 0.4 cm apart from another one of the electrodes.

In the embodiment of FIG. 11, the first patch portion 122 and the second patch portion 124 are formed from the same material as a unitary patch. As such, the first substrate 126 and the second substrate 128 are integrally formed from the same material. Some embodiments, however, may be differently configured. For example, some embodiments may be configured with separate first and second patches for supporting the first and second electrode arrays 130, 132.

Embodiments of a fetal and maternal monitoring system including the electrode set 120 of FIG. 11 may be configured to adaptively select at least one electrode 131, 133 to be used in a monopolar or bipolar arrangement in order to obtain the UA signal and/or the TAE signal. For example, the electrode 131, 133 or pair of electrodes 131, 133 used for generating the UA signal or the TAE signal may be selected, for example, based on the signal strength of the acquired signal and/or the signal-to-noise ratio. Some embodiments, however, may be differently configured. For example, a maternal and fetal monitoring system may use a predetermined electrode 131, 133 (or set of electrodes 131, 133) for acquiring the UA signals and a separate electrode 131, 133 (or set of electrodes131, 133) for acquiring the TAE signal.

FIG. 12 illustrates another embodiment of an electrode set 140 that includes a first electrode array 150 on a first patch portion 142 and a second electrode array 152 on a second patch portion 144. The first electrode array 150 includes a linear arrangement of sixteen individual electrodes 151 mounted on a first substrate 146 and arranged in a grid that is eight electrodes 151 wide and two electrodes 151 long. The sixteen electrodes 151 are spaced across the first patch portion 142 so that the first electrode array 150 may act as a low pass filter for acquiring the UA signal. The second electrode array 152 includes two electrodes 153 arranged linearly and mounted on a second substrate 148 of the second patch portion 144.

The electrodes in the first electrode array 150 may be configured in a monopolar arrangement with a reference electrode (not shown), where the output of a plurality of electrode is used to acquire the UA signals, as is described above. The outputs from electrodes or pairs of electrodes (which may be pairs of two electrodes in the electrode arrays 150 and 152 or may be output of each electrode paired to a reference electrode in a monopolar arrangement) are added together via hardware elements and/or logic gates, or digital signal processing, to generate the UA signal. The TAE signal is obtained from at least one of these electrodes 151 or 153 in a monopolar configuration, or from an output of a single pair from among the electrodes 151, 153. For example, the electrodes 151 in the second electrode array 152 may be utilized to obtain the TAE signal. The electrode(s) 151, 153 used to obtain the TAE signal may be used in a monopolar configuration by referencing a reference electrode, or as a pair of electrodes 151, 153 in a bipolar configuration.

The size, orientation, and/or arrangement of the individual electrodes 151, 153 in an array of electrodes 150, 152 may take on the various dimensions described above to create the desired filtering effect. As is described above, the filtering characteristics of the low pass electrode patch 142 of FIG. 12 may be based on the dimensions of the electrodes 151 in the first electrode array 150 and/or the materials with which the electrodes 151 are constructed. For example, the filtering characteristics of a low pass electrode patch 142 may be a function of the number of electrodes 151 in the electrode array 150, the spacing of the electrodes 151 in the array 150, the width of the electrode array 150, the height of the electrode array 150, the material(s) with which the electrodes 151 are constructed, the thickness of the electrodes 151, and/or the dimensions and materials of any additional components on the low pass electrode patch.

In the embodiment of FIG. 12, the first patch portion 142 and the second patch portion 144 are formed from the same material as a unitary patch. As such, the first substrate 146 and the second substrate 148 are integrally formed from the same material. Some embodiments, however, may be differently configured. For example, some embodiments may be configured with separate first and second patches for supporting the first and second electrode arrays 150, 152.

Embodiments of a fetal and maternal monitoring system including the electrode set 140 of FIG. 12 may be configured to adaptively select at least one electrode 151, 153 to be used in a monopolar or bipolar arrangement in order to obtain the UA signal and/or the TAE signal. For example, the electrode 151, 153 or pair of electrodes 151, 153 used for generating the UA signal or the TAE signal may be selected, for example, based on the signal strength of the acquired signal and/or the signal-to-noise ratio. Some embodiments, however, may be differently configured. For example, a maternal and fetal monitoring system may use a predetermined electrode 151, 153 (or set of electrodes 151, 153) for acquiring the UA signals and a separate electrode 151, 153 (or set of electrodes 151, 153) for acquiring the TAE signal.

FIG. 13 illustrates another arrangement of an electrode set 160 configured to obtain the low frequency UA signal and the TAE signal. Here, the electrode set 160 includes a single electrode patch 162 with a plurality of electrodes 171 arranged in a linear array and mounted on a substrate 166. The electrode patch 162 of FIG. 13 includes a total of eight individual electrodes 171 arranged in a linear array, i.e., a single line across the substrate 166. The electrodes 171 may be used in a monopolar configuration with reference to a reference electrode (not shown), or the electrodes 171 may be used in a bipolar configuration in which the electrodes 171 are referenced to one another in pairs, and the outputs of the pairs of electrodes are combined, as is described above. For example, electrodes in the subset 170 may be configured in a bipolar arrangement with reference electrode 172. Alternative pair arrangements are possible, such as referencing adjacent electrodes together. The linear array of electrodes 171 in the depicted example includes eight electrodes 171, but in other embodiments may include fewer electrodes (such as six electrodes) or more electrodes (such as 10 electrodes or up to 20 electrodes). In some implementations, it may be beneficial to include at least ten electrodes 171 in the linear array to provide sufficient electrode outputs to generate the desired low pass filtering effect. The TAE signal may be obtained via any of the electrodes, as described above.

Similar to the embodiments described above, the size, orientation, and/or spacing/arrangement of the individual electrodes 171 on the electrode patch 162 are configured to provide the appropriate low pass filtering effect. The filtering characteristics of the electrodes in the patch 162 may be based on the dimensions of the electrodes 171 and/or the materials with which the electrodes 171 are constructed. For example, the filtering characteristics of an electrode patch 162 may be a function of the number of electrodes 171, the spacing of the electrodes 171, the width of the arrangement of electrodes 171, the height of the arrangement of electrodes 171, the material(s) with which the electrodes 171 are constructed, the thickness of the electrodes 171, and/or the dimensions and materials of any additional components on the electrode patch.

Embodiments of a fetal and maternal monitoring system including the electrode set 160 of FIG. 13 may be configured to adaptively select at least one electrode 171 to be used in a monopolar or bipolar arrangement in order to obtain the UA signal and/or the TAE signal. For example, the electrode 171 (or pair of electrodes 171) used for generating the UA signal or the TAE signal may be selected, for example, based on the signal strength of the acquired signal and/or the signal-to-noise ratio. Some embodiments, however, may be differently configured. For example, a maternal and fetal monitoring system may use a predetermined electrode 171 (or set of electrodes 171) for acquiring the UA signals and a separate electrode 171 (or set of electrodes171) for acquiring the TAE signal.

FIG. 14 illustrates another embodiment of an electrode set 180 including an electrode patch 182 with a plurality of electrodes arranged in a grid. In the depicted example, the electrode patch 182 includes a total of thirty-two electrodes 191 mounted on a substrate 186 and arranged in a grid that is eight electrodes 191 wide and four electrodes 191 high. The UA signal is generated by combining (e.g., by summing or averaging) the outputs of electrodes, as described above. The electrode(s) 191 used to generate the UA signal may be adaptively selected by the maternal and fetal monitoring system, for example, based on the signal strength of the acquired UA signal and/or the signal-to-noise ratio. Additionally or alternatively, the electrodes utilized to generate the UA signal may be a predetermined set and arrangement of electrodes 191 on the electrode patch 182.

Similar to the embodiments described above, the size, orientation, and/or spacing/arrangement of the individual electrodes 191 on the electrode patch 182 are configured to provide the appropriate low pass filtering effect. The filtering characteristics of the electrodes in the patch 182 may be based on the dimensions of the electrodes 191 and/or the materials with which the electrodes 191 are constructed. For example, the filtering characteristics of an electrode patch 182 may be a function of the number of electrodes 191, the spacing of the electrodes 191, the width of the arrangement of electrodes 191, the height of the arrangement of electrodes 191, the material(s) with which the electrodes 191 are constructed, the thickness of the electrodes 191, and/or the dimensions and materials of any additional components on the electrode patch.

Embodiments of a fetal and maternal monitoring system including the electrode set 180 of FIG. 14 may be configured to adaptively select at least one electrode 191 to be used in a monopolar or bipolar arrangement to obtain the UA signal and/or the TAE signal. For example, the electrode 191 (or pair of electrodes 191) used for generating the UA signal or the TAE signal may be selected, for example, based on the signal strength of the acquired signal and/or the signal-to-noise ratio. Some embodiments, however, may be differently configured. For example, a maternal and fetal monitoring system may use a predetermined electrode 191 (or set of electrodes 191) for acquiring the UA signals and a separate electrode 191 (or set of electrodes 191) for acquiring the TAE signal.

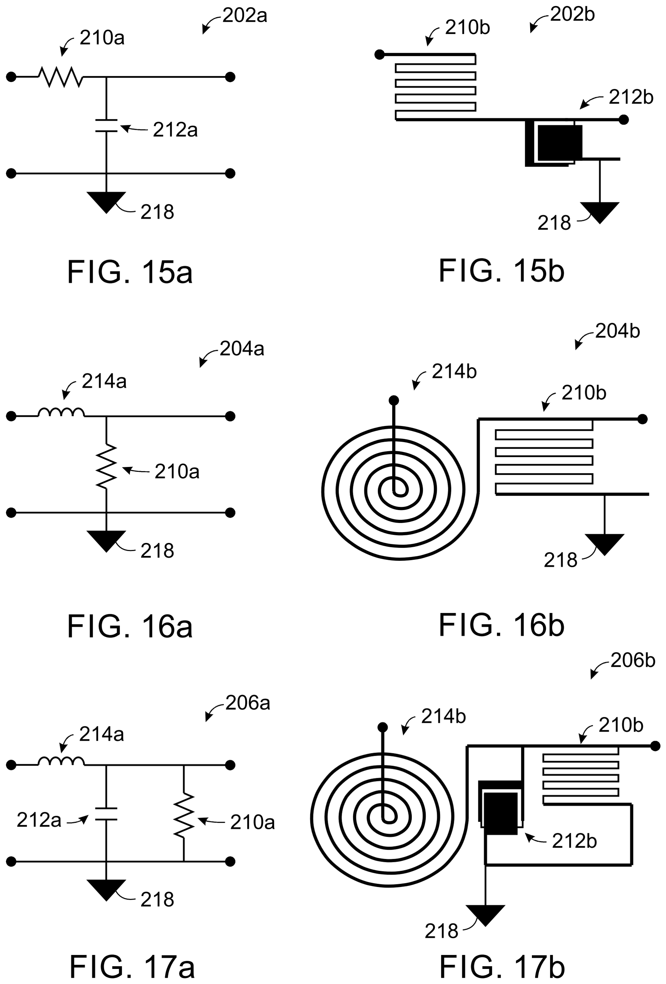

FIGS. 15A-17B show embodiments of electrical circuits for an electrode configured to provide low pass filtering to generate the UA signal. The electrodes utilized to generate the UA signal may include printed circuit elements, such as resistive and/or capacitive elements, to filter out the high frequency components and only pass the low frequency UA signal. FIG. 15A and 15B show an exemplary circuit 202 arrangement that may be included as part of an electrode to provide low pass filtering to signals obtained from said electrode. FIG. 15a schematically depicts the circuit 202a and FIG. 15B provides a top down view of a printed circuit 202b configured according to the schematic of FIG. 15A. The circuit 202a, 202b of FIGS. 15A and 15B include a resistor 210a, 210b, a capacitor 212a, 212b, and a ground connection 218. As illustrated in FIG. 15B, a printed circuit 202b may include at least one printed resistor 210b and/or at least one printed capacitor 212b. The printed elements 210b, 212b may be formed from a conductive ink that is printed onto a substrate, for example using an inkjet printing process.

FIG. 16A schematically depicts the circuit 204a and FIG. 16B provides a top down view of a printed circuit 204b configured according to the schematic of FIG. 16B. The circuit 204a, 204b of FIGS. 16A and 16B include a resistor 210a, 210b, an inductor 214a, 214b, and a ground connection 218. As illustrated in FIG. 16B, a printed circuit 204b may include at least one printed resistor 210b and/or at least one printed inductor 214b. The printed elements 210b, 214b may be formed from a conductive ink that is printed onto a substrate, for example using an inkjet printing process.

FIG. 17A schematically depicts the circuit 206a and FIG. 17B provides a top down view of a printed circuit 206b configured according to the schematic of FIG. 17A. The circuit 206a, 206b of FIGS. 17A and 17B include a resistor 210a, 210b, a capacitor 212a, 212b, an inductor 214a, 214b, and a ground connection 218. As illustrated in FIG. 17B, a printed circuit 202b may include at least one printed resistor 210b, at least one printed capacitor 212b, and/or at least one printed inductor 214b. The printed elements 210b, 212b, 214b may be formed from a conductive ink that is printed onto a substrate, for example using an inkjet printing process.

This written description uses examples to disclose the invention(s), including the best mode, and also to enable any person skilled in the art to make and use the invention(s). Certain terms have been used for brevity, clarity, and understanding. No unnecessary limitations are to be inferred therefrom beyond the requirement of the prior art because such terms are used for descriptive purposes only and are intended to be broadly construed. The patentable scope of the invention(s) is defined by the claims and may include other examples that occur to those skilled in the art. Such other examples are intended to be within the scope of the claims if they have features or structural elements that do not differ from the literal language of the claims, or if they include equivalent features or structural elements with insubstantial differences from the literal languages of the claims.

## Claims

1. A patient monitoring system configured to measure maternal electrophysiological signals, the system comprising:
a set of electrodes configured to obtain a uterine activity (UA) signal and a total abdominal electrical signal from an abdomen of a maternal patient, wherein the total abdominal electrical signal includes at least the UA signal, a maternal heart signal, and a fetal heart signal;
wherein at least a subset of the set of electrodes is configured as a low pass filter to obtain the UA signal; and
a patient monitor configured to:
receive the UA signal and the total abdominal electrical signal obtained by the set of electrodes;
subtract the UA signal from the total abdominal signal to generate a total heart signal containing at least the maternal heart signal and the fetal heart signal;
determine a maternal heart rate based on the total heart signal; and
output the UA signal and the maternal heart rate.

2. The system of claim 1, wherein at least the subset of the set of electrodes configured to act as the low pass filter is configured to only pass frequencies below a threshold frequency, wherein the threshold frequency is between 0.1 Hz and 2 Hz.

3. The system of claim 1, wherein at least one of the electrodes in the set of electrodes has a width of at least 15 cm such that it is configured to act as the low pass filter.

4. The system of claim 1, wherein at least one of the electrodes in the set of electrodes is a printed electrode and includes at least one printed circuit element configured to act as the low pass filter.

5. The system of claim 1, wherein the set of electrodes includes at least three electrodes comprising:
a first electrode configured in a monopolar arrangement to obtain the UA signal;
wherein the first electrode is configured to only pass frequencies below a threshold frequency; and
a pair of electrodes comprising a second electrode and a third electrode configured in a bipolar arrangement to obtain the total abdominal electrical signal from the maternal patient.

6. The system of claim 5, wherein the first electrode configured to obtain UA has a width of at least 10 cm.

7. The system of claim 5, wherein the first electrode has a width of at least 15 cm.

8. The system of claim 5, wherein the first electrode has a width of at least 15 cm and wherein the second electrode and the third electrode has a width less than 2 cm.

9. The system of claim 5, wherein the first electrode, the second electrode, and the third electrode are mounted on a substrate comprising a patch configured to adhere to the abdomen of the maternal patient.

10. The system of claim 9, wherein the patch is configured such that the second electrode and the third electrode are movable with respect to the first electrode.

11. The system of claim 1, wherein the set of electrodes is an electrode array mounted on a substrate comprising a patch configured to adhere to the abdomen of the maternal patient, wherein the electrode array is configured to output multiple signals that are utilized together to generate the UA signal.

12. The system of claim 11, wherein the electrode array comprises at least one linear array of at least 4 electrodes arranged in a line.

13. The system of claim 11, wherein the electrode array comprises a grid, wherein the grid is at least 6 electrodes wide and at least 3 electrodes high.

14. The system of claim 11, wherein each electrode in the electrode array is separated by at least 4 mm.

15. The system of claim 1, further comprising an ultrasound transducer configured to be adhered to the maternal abdomen and to obtain the fetal heart signal; and
wherein the patient monitor is configured to determine the maternal heart rate by subtracting the fetal heart signal from the total heart signal.
